# EUROPEAN PATENT SPECIFICATION

(11) **EP 3 207 877 B1**
(45) Date of publication and mention of the grant of the patent: **31.03.2021**
(21) Application number: 15851013.1
(22) Date of filing: 14.10.2015
(51) Int. Cl.: A61B 8/08, G01S 7/52

(54) **ULTRASONIC DIAGNOSIS DEVICE**
ULTRASCHALLDIAGNOSEVORRICHTUNG
DISPOSITIF DE DIAGNOSTIC À ULTRASONS

(30) Priority: 16.10.2014 JP 2014211882
(43) Date of publication of application: 23.08.2017
(73) Proprietor: Hitachi, Ltd., Chiyoda-ku, Tokyo 100-8280 (JP)
(72) Inventor: SONOYAMA, Teruyuki, Mitaka-shi Tokyo 181-8622 (JP); INOUE, Noriaki, Mitaka-shi Tokyo 181-8622 (JP)
(74) Representative: Strehl Schübel-Hopf & Partner
(86) International application number: PCT/JP2015/078984
(87) International publication number: WO 2016/060146

(56) References cited:
- WO-A1-2011/052400
- WO-A1-2013/153968
- WO-A1-2014/061810
- JP-A- 2007 090 003
- JP-A- 2014 000 260
- JP-A- 2015 128 554
- US-A1- 2010 185 090
- US-A1- 2012 203 108
- US-A1- 2013 296 698

## Description

### TECHNICAL FIELD

The present invention relates to an ultrasonic diagnosis device, and more particularly to a technology that measures a displacement of a tissue.

### BACKGROUND

There is known an ultrasonic diagnosis device that measures a displacement of a tissue within a subject and obtains diagnosis information from within the subject. For example, it transmits an ultrasonic wave to generate a shear wave within the subject, measures a displacement of a tissue accompanying the shear wave propagation by the ultrasonic wave, and can obtain diagnosis information such as hardness of a tissue within the subject on the basis of a propagation velocity and the like of the shear wave.

For example, Patent Document 1 discloses an invention that measures a displacement of a shear wave at a plurality of positions different from one another, and calculates a propagation velocity of the shear wave at each position on the basis of a time when a maximum displacement was obtained.
The document US 2010/185090 discloses an ultrasonic inspection device which generates a time-space map of displacement data. The document US2013/296698 discloses an ultrasonic inspection device which generates an "image filed" and, based thereon, a velocity map and to apply filtering to eliminate high-frequency noise.
The document US 2012/203108 A1 discloses an ultrasonic inspection device which seeks to eliminate the influence on unintentional hand movement from displacement data.
The document JP2007 090003 A discloses an ultrasonic inspection device which is configured to detect a noise peak in the power spectrum of a cardiac cycle and to remove the noise peak with correction unit constituted by a band pass filter where necessary.
The documents WO 2014/061810 A1 and US 2015/216508 A1 disclose an ultrasonic inspection device which is configured to generate data (Doppler data) obtained by extracting moving member information such as a velocity, a dispersion, a power, and the like that are under the influence of the Doppler effect, for a plurality of points.
The document WO 2013/153968 A1 discloses an ultrasonic inspection device configured to eliminate the frequency component by camera shaking of a heart rate of an operator.

### CITATION LIST

### PATENT LITERATURE

Patent Document 1: USP 8,118,744

### SUMMARY

### TECHNICAL PROBLEM

In view of the above background technologies, the inventors of this application have carried out repeated research and development into a technology to measure a displacement of a tissue by an ultrasonic diagnosis device. In the process of the research and development, the inventors of this application have noticed that the movement of minute blood vessels and bloodstreams within the subject affects the measurement of the displacement of the tissue.

For example, it was found that when a propagation velocity of a shear wave is measured, a displacement of a tissue fluctuates periodically due to movements of minute blood vessels and bloodstreams, and the measurement of the propagation velocity is affected. Specially, there are minute blood vessels that are not drawn by the functions of color Doppler or the like, and it is quite difficult to measure the shear wave while avoiding such minute blood vessels.

The present invention was achieved in the process of the above-described research and development, and its purpose is to detect a periodic displacement in measurement of a displacement of a tissue using an ultrasonic wave.

### SOLUTION TO PROBLEMS

A preferable ultrasonic diagnosis device suited to the above object is defined in the independent claim 1.

The periodic displacement is detected by the above device on the basis of the measured displacement of the tissue. Therefore, it becomes possible to specify the displacement which fluctuates periodically due to, for example, the movement of minute blood vessels or bloodstreams.

The ultrasonic diagnosis device transmits a push wave of an ultrasonic wave for generating a shear wave through the subject and for transmitting a tracking wave of an ultrasonic wave to the subject, the measurement unit measures a displacement of the tissue after the generation of the shear wave on the basis of the reception signal of the ultrasonic wave which is obtained by the tracking wave, and the detection unit detects the periodic displacement after the generation of the shear wave.

The ultrasonic diagnosis device obtains velocity information of the shear wave in the subject by using the displacement measurement result after the generation of the shear wave measured by the measurement unit, whose the periodic displacement detected by the detection unit is corrected to be lowered and removed.

According to a desired specific example, the measurement unit measures, at a plurality of points in the subject, the displacement of the tissue at the individual points on the basis of the reception signal at each point, and generates displacement data showing the displacement measured at the plurality of points over a plurality of times, and the detection unit, on the basis of the displacement data, detects a periodic displacement among the displacements measured at the plurality of points.

The measurement unit generates displacement data that show a time along one axis and positions of a plurality of points along the other axis.

According to a desired specific example, the detection unit performs frequency analysis of a temporal change of displacement at each point on the basis of the displacement data, and if a maximum frequency component satisfying judgment conditions is contained, it is judged that the displacement at the point is periodic.

According to a desired specific example, the ultrasonic diagnosis device forms a display image which shows displacements at a plurality of points over a plurality of times on the basis of the displacement data generated by the measurement unit.

According to a desired specific example, the ultrasonic diagnosis device forms a display image which specifies the individual points where the periodic displacements were detected by the detection unit among the plurality of points where the displacements were measured by the measurement unit.

### ADVANTAGEOUS EFFECTS OF INVENTION

The periodic displacement can be detected by measuring a displacement of a tissue using an ultrasonic wave by the present invention. For example, according to a preferable embodiment of the invention, it becomes possible to specify a displacement that fluctuates periodically due to movement of minute blood vessels and bloodstreams.

### BRIEF DESCRIPTION OF DRAWINGS

FIG. 1 is a diagram showing the overall structure of a preferable ultrasonic diagnosis device in implementation of the invention;
FIG. 2 is a diagram illustrating a specific example of measuring generation and displacement of a shear wave;
FIG. 3 is a diagram showing a specific example of a time-space map;
FIG. 4 is a diagram showing a specific example of fluctuation;
FIG. 5 is a diagram illustrating detection of fluctuation;
FIG. 6 is a diagram showing an example of calculating a propagation velocity of a shear wave; and
FIG. 7 is a diagram showing a specific example of a display image.

### DESCRIPTION OF EMBODIMENTS

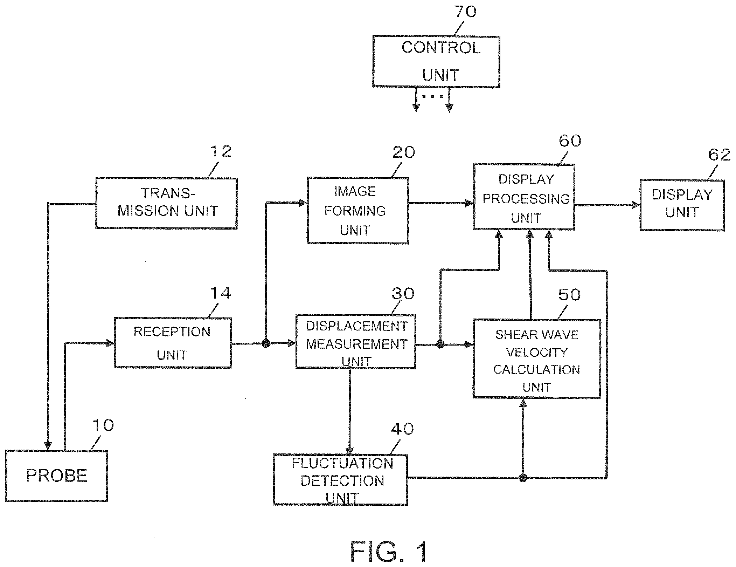

FIG. 1 is a diagram showing the overall structure of a preferable ultrasonic diagnosis device in implementation of the invention. A probe 10 is an ultrasonic probe which transmits/receives an ultrasonic wave to/from a tissue in a subject (living body), for example, an area including a diagnosis subject such as an organ. The probe 10 has a plurality of vibration elements that individually transmit/receive or transmit an ultrasonic wave, and the plurality of vibration elements are controlled for transmission by a transmission unit 12 to form a transmission beam.

Also, the plurality of vibration elements equipped by the probe 10 receive the ultrasonic wave from the area including the diagnosis subject, the signal thus obtained is output to a reception unit 14, and the reception unit 14 forms a reception beam to collect a reception signal (echo data) along the reception beam. Further, the probe 10 is desirably, for example, a convex type but may be a linear type or the like.

The probe 10 has a function to transmit an ultrasonic wave (push wave) for generating a shear wave in an area including a tissue which becomes a diagnosis subject, a function to transmit/receive an ultrasonic wave (tracking wave) for measuring a displacement of the tissue accompanying the shear wave, and a function to transmit/receive an image forming ultrasonic wave.

Transmission of the ultrasonic wave is controlled by the transmission unit 12 will now be described. When the shear wave is generated, the transmission unit 12 outputs a push wave transmission signal to the plural vibration elements which are equipped in the probe 10, thereby forming a push wave transmission beam. Also, when the shear wave is measured, the transmission unit 12 outputs a tracking wave transmission signal to the plurality of vibration elements which are equipped in the probe 10, thereby forming a tracking wave transmission beam. Further, when an ultrasonic image is formed, the transmission unit 12 outputs an image forming transmission signal to the plurality of vibration elements which are equipped in the probe 10, and the image forming transmission beam is scanned.

Based on the received wave signals obtained from the plurality of vibration elements when the probe 10 transmits/receives the tracking wave, the reception unit 14 forms the reception beam of the tracking wave, and obtains a reception signal corresponding to the reception beam. In addition, based on the received wave signals obtained from the plurality of vibration elements when the probe 10 transmits/receives an image forming ultrasonic wave, the reception unit 14 forms an image forming reception beam and generates a reception signal corresponding to the reception beam.

The image forming ultrasonic beam (transmission beam and reception beam) is scanned in a two-dimensional plane including a diagnosis subject, and image forming reception signals are collected from the two-dimensional plane. The image forming ultrasonic beam may naturally be scanned three-dimensionally in a three-dimensional space to collect the image forming reception signals from the three-dimensional space.

An image forming unit 20 forms image data of the ultrasonic wave on the basis of the image forming reception signal collected by the reception unit 14. The image forming unit 20 forms, for example, image data of a B-mode image (tomographic image) of an area including tissue of an organ or the like which is a diagnosis subject. Also, when the image forming reception signals are being collected three-dimensionally, the image forming unit 20 may form image data of a three-dimensional ultrasonic image.

A displacement measurement unit 30 measures a displacement of a tissue on the basis of the reception signal corresponding to the reception beam of the tracking wave obtained from the reception unit 14 after generation of the shear wave inside the subject. A fluctuation detection unit 40 detects a periodic displacement on the basis of the displacement measurement result obtained from the displacement measurement unit 30. A shear wave velocity calculation unit 50 calculates the propagation velocity of the shear wave inside the subject on the basis of the measurement result obtained from the displacement measurement unit 30 and the detection result obtained from the fluctuation detection unit 40. Processing by the displacement measurement unit 30, the fluctuation detection unit 40, and the shear wave velocity calculation unit 50 will be described later in detail.

A display processing unit 60 forms a display image on the basis of the image data of the ultrasonic image obtained from the image forming unit 20, the velocity information obtained by the shear wave velocity calculation unit 50, the measurement result obtained from the displacement measurement unit 30, and the detection result obtained from the fluctuation detection unit 40. The display image formed by the display processing unit 60 is displayed on a display unit 62.

A control unit 70 performs overall control of the inside of the ultrasonic diagnosis device shown in FIG. 1. The ultrasonic diagnosis device in FIG. 1 is desirably equipped with, for example, an operation device which is comprised of a mouse, a keyboard, a track ball device, a touch panel, and other switches. In addition, the overall control by the control unit 70 reflects also an instruction accepted from a user through the operation device.

Among the structures (individual function blocks with reference numerals) shown in FIG. 1, the transmission unit 12, the reception unit 14, the image forming unit 20, the displacement measurement unit 30, the fluctuation detection unit 40, the shear wave velocity calculation unit 50, and the display processing unit 60 each can be realized using, for example, hardware such as an electric/electronic circuit and a processor, and if necessary, a device such as a memory may be used for the realization. Also, the functions corresponding to the individual units may be realized by cooperation between hardware such as a CPU or a processor and a memory, and software (a program) which regulates the operation of the CPU or the processor. A preferable specific example of the display unit 62 is a liquid crystal display or the like. The control unit 70 can also be realized by, for example, cooperation between hardware such as a CPU or a processor and a memory, and software (a program) which regulates the operation of the CPU or the processor.

The overall structure of the ultrasonic diagnosis device of FIG. 1 is as described above. Next, generation of the shear wave and measurement of displacement by the ultrasonic diagnosis device of FIG. 1 are described in detail. Also, for the individual structures (individual function blocks) shown in FIG. 1, reference numerals in FIG. 1 are used in the following description.

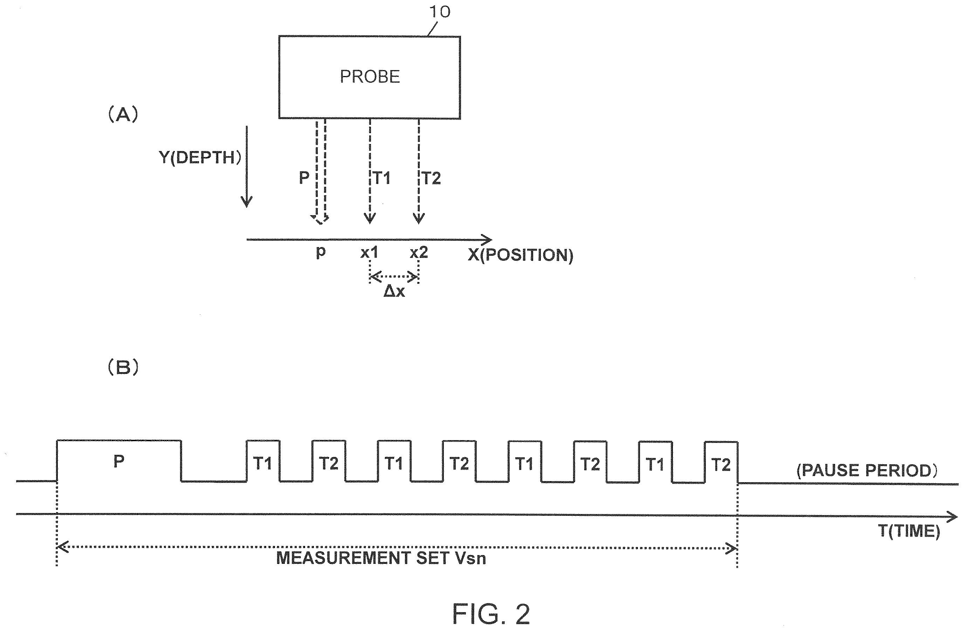

FIG. 2 is a diagram illustrating a specific example of generation of a shear wave and measurement of displacement. FIG. 2(A) illustrates a specific example of a transmission beam P of a push wave formed using the probe 10, and ultrasonic beams T1, T2 of the tracking wave.

In FIG. 2(A), the transmission beam P of the push wave is formed along a direction of depth Y to pass through the position p in X direction. For example, the transmission beam P of the push wave is formed with the position p on the X-axis shown in FIG. 2(A) as a focal point. For example, the position p is set as a desired position by a user (tester) such as a doctor who has confirmed an ultrasonic image on a diagnosis subject within a living body shown on the display unit 62.

When the transmission beam P is formed with the position p as the focal point and the push wave is transmitted, a relatively strong shear wave is generated at the position p and near it in the living body. FIG. 2(A) shows a specific example of measuring a propagation velocity in the X direction of the shear wave which is generated at the position p.

In the specific example of FIG. 2(A), two ultrasonic beams T1, T2 of the tracking wave are formed. The ultrasonic beam (transmission beam and reception beam) T1 is formed to pass through a position x1 on the X-axis shown in, for example, FIG. 2(A), and the ultrasonic beam (transmission beam and reception beam) T2 is formed to pass through a position x2 on the X-axis shown in, for example, FIG. 2(A). The position x1 and the position x2 may be set, for example, at desired positions by a user who has confirmed the ultrasonic image of the diagnosis subject displayed on the display unit 62, and the ultrasonic diagnosis device of FIG. 1 may set the position x1 and the position x2 at points away from the position p by a prescribed distance along the X direction.

FIG. 2(B) shows a specific example of generation timing of the transmission beam P of the push wave and the ultrasonic beams T1, T2 of the tracking wave. The horizontal axis in FIG. 2(B) is a time axis t.

In FIG. 2(B), the period P is a period, in which the transmission beam P of the push wave is formed, and the periods T1, T2 are periods in which the respective ultrasonic beams T1, T2 of the tracking wave are formed.

In the period P, a push wave of multiple waves is transmitted. For example, an ultrasonic wave of a continuous wave is transmitted in the period P. And, a shear wave is generated at, for example, the position p just after the period P has expired.

In the periods T1, T2, so-called tracking waves of pulse waves of approximately one to several waves are transmitted, and a reflected wave accompanying the pulse wave is received. For example, the ultrasonic beams T1, T2 passing through the positions x1, x2 are formed, and reception signals are obtained at a plurality of depths including the positions x1, x2. In other words, reception signals are obtained from the plurality of depths for each of the ultrasonic beams T1, T2.

The tracking wave is transmitted/received repeatedly over the plurality of periods. That is to say, as shown in FIG. 2(B), the periods T1, T2 are alternately repeated until, for example, a displacement of a tissue accompanying the shear wave is confirmed.

The displacement measurement unit 30 forms a time-space map related to the ultrasonic beam T1 on the basis of the reception signal of the ultrasonic beam T1 of the tracking wave, and forms a time-space map related to the ultrasonic beam T2 on the basis of the reception signal of the ultrasonic beam T2 of the tracking wave.

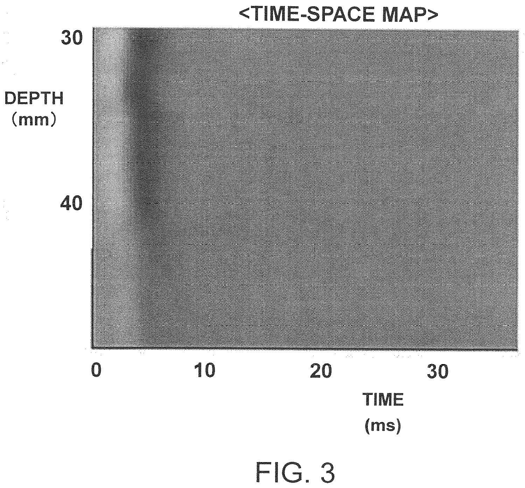

FIG. 3 is a diagram showing a specific example of the time-space map. The displacement measurement unit 30 calculates a phase displacement of the reception signal at a plurality of depths (a plurality of points in a depth direction) on the basis of the reception signal of the ultrasonic beam T1 of the tracking wave. The displacement measurement unit 30 calculates a phase displacement (a differential value of a phase) of the reception signal at each depth over a plurality of times. Moreover, the displacement measurement unit 30 forms a time-space map with the horizontal axis defined as a time (time axis) and the vertical axis as a depth by mapping the phase displacement of the reception signal.

The specific example of the time-space map illustrated in FIG. 3 shows the phase displacement of the reception signal with brightness in the time-space map. For example, it is determined that brightness is higher (white) when the phase displacement is in a positive direction and the absolute value is larger, and brightness is lower (black) when the phase displacement is in a negative direction and the absolute value is larger. In the specific example of FIG. 3, the phase displacement changes relatively largely from high brightness (white) to low brightness (black) in a period from time 0 (zero) to 10 ms (milliseconds), in which the shear wave passes through.

Also, the time-space map in FIG. 3 is merely one of specific examples, and the phase displacement of the reception signal may be expressed by a display mode other than brightness, such as a color. For example, the color may be based on red when the phase displacement is in a positive direction and the absolute value is larger, the color may be based on green when the phase displacement is close to zero, and the color may be based on blue when the phase displacement is in a negative direction and the absolute value is larger.

Thus, the displacement measurement unit 30 forms a time-space map related to the ultrasonic beam T1 based on the reception signal of the ultrasonic beam T1 of the tracking wave. In addition, the displacement measurement unit 30 calculates a phase displacement of the reception signal at a plurality of depths on the basis of the reception signal of the ultrasonic beam T2 of the tracking wave, and forms a time-space map related to the ultrasonic beam T2.

Returning to FIG. 2, the shear wave velocity calculation unit 50 calculates a propagation velocity Vs in the X-axis direction of the shear wave on the basis of the phase displacement at the position x1 and the position x2 which are varied by an influence of a shear wave generated at the position p. For example, the propagation velocity Vs=Δx/(t2-t1) in the X-axis direction of the shear wave is calculated on the basis of a time t1 when a phase displacement at the position x1 becomes maximum, a time t2 when a phase displacement at the position x2 becomes maximum, and a distance Δx between the position x1 and the position x2. In addition, the propagation velocity of the shear wave may be calculated by another known technique. Further, based on the propagation velocity of the shear wave, elasticity information such as the elasticity value or the like of the tissue with the shear wave measured may be calculated, and a viscosity parameter, attenuation, frequency characteristics, etc. may be derived as tissue information.

The measurement set Vsn shown in FIG. 2(B) is a period from the start of the push wave transmission to the calculation of the propagation velocity of the shear wave. After the measurement set Vsn is over, it is desirable to provide a pause period in which the probe 10 is cleaned. The following measurement set Vsn may be additionally started after the pause period.

Also, in the specific example of FIG. 2, the ultrasonic beams T1, T2 of the tracking wave are formed toward the positive direction side of the X-axis with respect to the transmission beam P of the push wave, but the shear wave which propagates to the negative direction side of the X-axis may be measured by forming the ultrasonic beams T1, T2 of the tracking wave toward the negative direction side of the X-axis with respect to the transmission beam P of the push wave. Of course, it is desirable that the position p of the transmission beam P of the push wave and the positions x1, x2 of the ultrasonic beams T1, T2 of the tracking wave are appropriately set according to the diagnosis subject, diagnostic conditions, etc.

Incidentally, when the propagation velocity of the shear wave is measured, a displacement of a tissue fluctuates periodically due to movement of minute blood vessels and bloodstreams within a measurement region (region of interest), and this periodic fluctuation might affect the measurement of the propagation velocity of the shear wave.

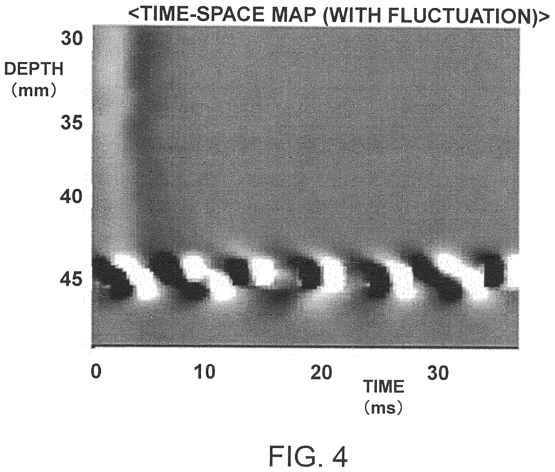

FIG. 4 is a diagram showing a specific example of fluctuation. In FIG. 4, a specific example of a time-space map which is obtained when fluctuation occurs is shown. In the time-space map shown in FIG. 4 compared with the time-space map shown in FIG. 3, the fluctuation occurs near a depth of 45 mm (millimeters). In other words, the phase displacement of the reception signal repeats periodically low brightness (black) and high brightness (white) at a depth of near 45 mm over a relatively long period (0-30 ms or more), and the phase displacement fluctuates periodically.

Therefore, at the depth of near 45 mm, it is difficult to specify a change of phase displacement associated with the passage of the shear wave, and the propagation velocity of the shear wave cannot be measured. Even if the propagation velocity of the shear wave could be measured in a region (depth) where fluctuation has occurred, reliability of the measurement result would be doubtful.

Then, the fluctuation detection unit 40 detects fluctuation which is a periodic displacement on the basis of the displacement measurement result by the displacement measurement unit 30.

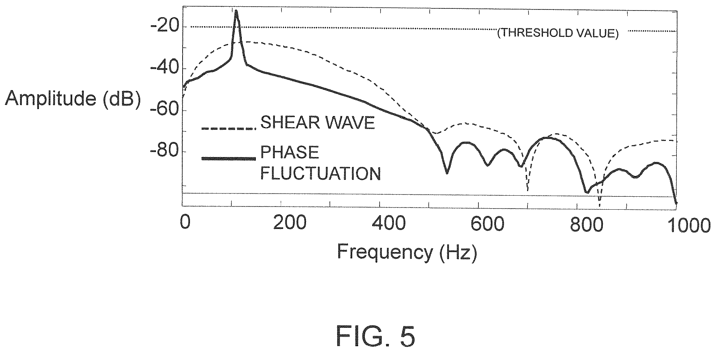

FIG. 5 is a diagram illustrating a specific example of fluctuation detection. The fluctuation detection unit 40 performs frequency analysis of a change in phase displacement at individual depths with time on the basis of the time-space map obtained from the displacement measurement unit 30, and checks whether there is a frequency component corresponding to the fluctuation.

FIG. 5 illustrates a result of frequency analysis of a temporal change of phase displacement. In FIG. 5, the horizontal axis shows a frequency (Hz: Hertz), and the vertical axis shows the power spectrum intensity; namely, the intensity of each frequency component (dB: decibel).

FIG. 5 shows a frequency spectrum (solid line) of "phase fluctuation" at a depth where fluctuation occurs, and a frequency spectrum (broken line) of "shear wave" at a depth where fluctuation does not occur.

The frequency spectrum of the "phase fluctuation" shows a peak (maximum) with a prominent intensity at a particular frequency, or near 100 Hz in the specific example of FIG. 5. On the other hand, a prominent peak like the "phase fluctuation" does not appear in a frequency spectrum of the "shear wave" not including a fluctuation. Therefore, when there is a peak (maximum) with a prominent intensity in the frequency spectrum of a phase change at each depth, the fluctuation detection unit 40 judges that displacement at that depth is periodical and the fluctuation has occurred at that depth. When there is a peak of intensity exceeding a threshold value in, for example, the frequency spectrum of the phase change at each depth, the fluctuation detection unit 40 judges that the fluctuation has occurred at that depth.

In addition, the fluctuation detection unit 40 may detect fluctuation by processing different from the frequency analysis. For example, within the time-space map, the absolute value of the phase displacement at each depth over a plurality of times may be added, and a depth where fluctuation has occurred may be specified on the basis of the addition result obtained at each depth. As exemplified in FIG. 4, at the depth where the fluctuation has occurred, the phase displacement of the reception signal varies periodically over a relatively long period, and the addition result of the absolute value of the phase displacement becomes relatively large, but at a depth where fluctuation has not occurred inversely, the period in which the phase displacement of the reception signal becomes 0 (zero) is dominant and the addition result of the absolute value of the phase displacement becomes relatively small. Then, the fluctuation detection unit 40 adds, for example, the absolute value of the phase displacement at each depth over a plurality of times, and when the addition result obtained at each depth exceeds a determination threshold value, it may be judged that the fluctuation has occurred at that depth. An image portion (depth) where fluctuation has occurred may be judged by image analysis processing of the time-space map.

The fluctuation detection unit 40 detects a depth where fluctuation occurs in each of the time-space map of the ultrasonic beam T1 and the time-space map of the ultrasonic beam T2. The shear wave velocity calculation unit 50 reduces from the time-space map, which is a measurement result obtained from the displacement measurement unit 30, the periodic displacement fluctuation detected by the fluctuation detection unit 40, desirably removes the fluctuation completely, and calculates the propagation velocity of the shear wave.

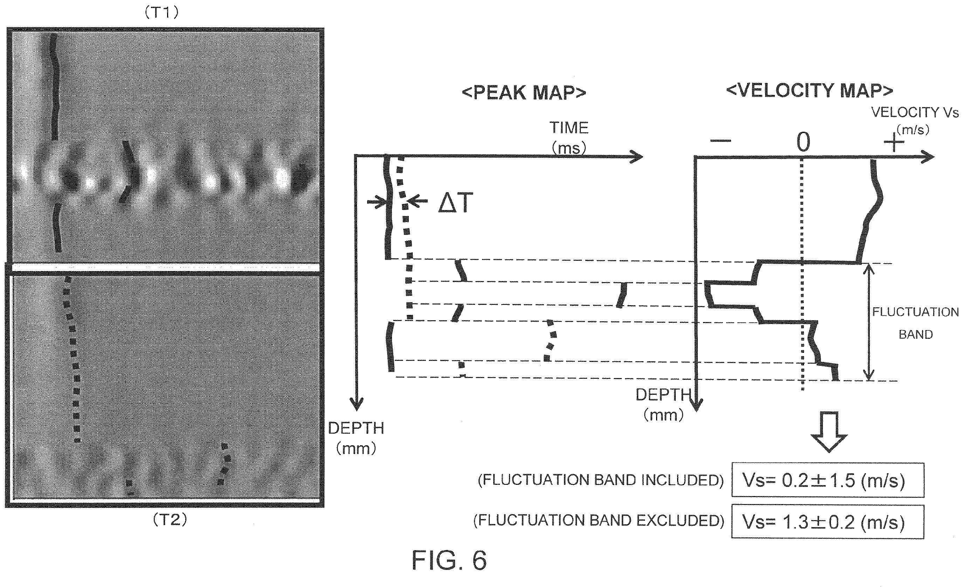

FIG. 6 is a diagram illustrating examples of calculating propagation velocities of shear waves. FIG. 6 shows a time-space map (T1) of the ultrasonic beam T1 and a time-space map (T2) of the ultrasonic beam T2.

The shear wave velocity calculation unit 50 specifies a peak of the phase displacement within the time-space map. For example, the shear wave velocity calculation unit 50 specifies a time when the phase displacement becomes maximum at each depth within the time-space map, and specifies a peak time when the phase displacement becomes maximum at a plurality of depths.

In the specific example shown in FIG. 6, the solid line in the time-space map (T1) is a trace line showing a peak time, and the broken line in the time-space map (T2) is a trace line showing a peak time.

Moreover, the peak map shown in FIG. 6 has the trace line (solid line) on the time-space map (T1) and the trace line (broken line) on the time-space map (T2) overlapped mutually with the time axes aligned.

The shear wave velocity calculation unit 50 calculates a time difference ΔT between a peak time within the time-space map (T1) and a peak time within the time-space map (T2) at each depth, and calculates a shear wave propagation velocity Vs=Δx/ΔT at each depth on the basis of the time difference ΔT. The Δx is a distance between the ultrasonic beam T1 and the ultrasonic beam T2 at each depth.

The velocity map shown in FIG. 6 shows the shear wave propagation velocities Vs at a plurality of depths which are calculated on the basis of the time-space map (T1) and the time-space map (T2). In the velocity map of FIG. 6, the vertical axis is a depth and the horizontal axis is the propagation velocity Vs.

The shear wave velocity calculation unit 50 calculates a statistical propagation velocity Vs within the time-space map on the basis of the propagation velocity Vs at a plurality of depths. For example, "average value±standard deviation" at a plurality of depths is calculated as the statistical propagation velocity Vs. In the specific example in FIG. 6, the statistical propagation velocity Vs=0.2±1.5 (m/s) which is obtained from the overall depth including a fluctuation band and the statistical propagation velocity Vs=1.3±0.2 (m/s) obtained from the remaining depth excluding the fluctuation band are calculated.

The fluctuation band is a region (fluctuation portion) which comprises the plurality of depths at which fluctuation was detected in at least one of the time-space map (T1) and the time-space map (T2). Within the fluctuation band, the phase displacement is affected by the fluctuation, and a peak of the phase displacement associated with the shear wave cannot be specified properly. For example, in the specific example of FIG. 6, the shear wave propagates from the ultrasonic beam T1 side toward the ultrasonic beam T2 side, so that the propagation velocity Vs of the shear wave should become positive (+), but in the fluctuation band, a peak of the phase displacement associated with the shear wave is not specified properly, so that there is obtained a calculation result that the propagation velocity Vs becomes negative (-). Therefore, an improper calculation result in the fluctuation band is also reflected in the statistical propagation velocity Vs including the fluctuation band.

On the other hand, an improper calculation result in the fluctuation band is not reflected to the statistical propagation velocity Vs with the fluctuation band excluded, and the statistical propagation velocity Vs can be obtained at a plurality of depths where the peak of the phase displacement associated with the shear wave could be specified properly. Therefore, it becomes possible to obtain a high-precision and stable measurement result at the statistical propagation velocity Vs with the fluctuation band excluded.

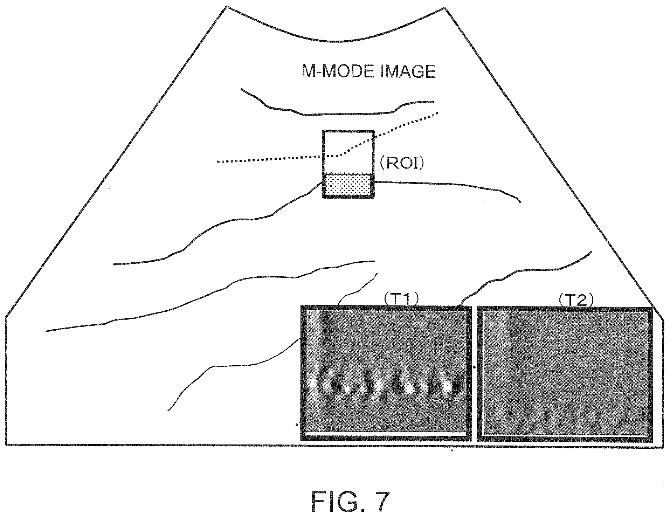

FIG. 7 is a diagram showing a specific example of a display image. FIG. 7 shows a specific example of a display image which is formed by the display processing unit 60 and shown on the display unit 62. The display image of FIG. 7 is formed on the basis of a B-mode image (tomographic image) which is formed by the image forming unit 20 and two time-space maps (T1, T2) which are formed by the displacement measurement unit 30, and displayed on the display unit 62 in, for example, a pause period (see FIG. 2).

In the B-mode image, a region of interest (ROI) may be displayed. For example, a rectangular mark indicating the region of interest (ROI) is displayed similar to the specific example illustrated in FIG. 7. The region of interest (ROI) is a region where the shear wave was measured; namely, a region where two time-space maps (T1, T2) were obtained.

In addition, the region of interest (ROI) specifies therein a region corresponding to a fluctuation portion (fluctuation band in FIG. 6) detected by the fluctuation detection unit 40. For example, the fluctuation portion is highlighted in display modes such as a pattern, brightness, and colors within the region of interest (ROI). Thus, for example, when a fluctuation portion is large (broad) within the region of interest (ROI), the user may reset the position of the region of interest (ROI).

Also, in the specific example of FIG. 7, two time-space maps (T1, T2) are shown on the B-mode image, but the two time-space maps (T1, T2) may be shown not to overlap the B-mode image and, for example, display and non-display may be switched according to an instruction from the user. In addition, the calculation result of the propagation velocity Vs calculated by the shear wave velocity calculation unit 50, for example, the statistical propagation velocity Vs (FIG. 6) excluding a fluctuation band, may be displayed by numerical values. As reference information, the calculation result of the statistical propagation velocity Vs (FIG. 6) including the fluctuation band may be displayed.

While preferable embodiments of the present invention have been described, the above-described embodiments are mere examples in all respects and do not limit the scope of the invention.

### REFERENCE SIGNS LIST

10: Probe, 12: Transmission unit, 14: Reception unit, 20: Image forming unit, 30: Displacement measurement unit, 40: Fluctuation detection unit, 50: Shear wave velocity calculation unit, 60: Display processing unit, 62: Display unit, 70: Control unit

## Claims

1. An ultrasonic diagnosis device, comprising:
a transmission/reception unit (10, 12, 14) which is configured to output a transmission signal of an ultrasonic wave and to obtain a reception signal of the ultrasonic wave from a subject,
a measurement unit (30) which is configured to measure a displacement of a tissue in the subject on the basis of the reception signal of the ultrasonic wave, and
a fluctuation detection unit (40) which is configured to detect a periodic displacement on the basis of the measured displacement of the tissue, wherein:
the ultrasonic diagnosis device is configured to transmit a push wave of an ultrasonic wave for generating a shear wave through the subject and to transmit a tracking wave of an ultrasonic wave to the subject,
the measurement unit (30) is configured to measure a phase displacement of the tissue after the generation of the shear wave on the basis of the reception signal of the ultrasonic wave which is obtained by the tracking wave, and
the fluctuation detection unit (40) is configured to detect the periodic displacement after the generation of the shear wave,
wherein
the ultrasonic diagnosis device includes a shear wave velocity calculation unit (50) configured to obtain a statistical propagation velocity (Vs) of the shear wave in the subject by using the displacement measurement result at a plurality of depths after the generation of the shear wave measured by the measurement unit (30) and the detection result obtained from the fluctuation detection unit and further wherein the ultrasonic diagnosis device is configured to:
- generate, as a time-space map, phase displacement data that show a time along one axis and positions of the plurality of depths along the other axis;
- determine a fluctuation band as a region which comprises a plurality of positions at which fluctuation was detected in the time-space map, wherein the phase displacement is affected by the fluctuation in the fluctuation band, and
- obtain the statistical propagation velocity (Vs) from the displacement measurement result at the plurality of depths excluding the fluctuation band.

2. The ultrasonic diagnosis device according to Claim 1, wherein:
the measurement unit (30) is configured to measure, at a plurality of points in the subject, the displacement of the tissue at the individual points on the basis of the reception signal at each point, and to generate displacement data showing the displacement measured at the plurality of points over a plurality of times, and
the fluctuation detection unit (40), on the basis of the displacement data, is configured to detect a periodic displacement among the displacements measured at the plurality of points.

3. The ultrasonic diagnosis device according to Claim 2, wherein:
the fluctuation detection unit (40) is configured to perform frequency analysis of a temporal change of displacement at each point on the basis of the displacement data, and if a maximum frequency component satisfying judgment conditions is contained, to judge that the displacement at the point is periodic.

4. The ultrasonic diagnosis device according to Claim 2 or 3, wherein:
the ultrasonic diagnosis device is configured to form a display image which shows displacements at a plurality of points over a plurality of times on the basis of the displacement data generated by the measurement unit (30).

5. The ultrasonic diagnosis device according to Claim 2, wherein:
the ultrasonic diagnosis device is configured to form a display image which specifies the individual points where the periodic displacements were detected by the fluctuation detection unit among the plurality of points where the displacements were measured by the measurement unit (30).

## Patentansprüche

1. Ultraschall-Diagnosevorrichtung, umfassend:
eine Sende-/Empfangseinheit (10, 12, 14), die dazu ausgelegt ist, ein Sendesignal einer Ultraschallwelle auszugeben und ein Empfangssignal der Ultraschallwelle von einem Subjekt zu empfangen,
eine Messeinheit (30), die dazu ausgelegt ist, eine Verschiebung eines Gewebes in dem Subjekt auf der Basis des Empfangssignals der Ultraschallwelle zu messen, und
eine Schwankungsdetektionseinheit (40), die dazu ausgelegt ist, eine periodische Verschiebung auf der Basis der gemessenen Verschiebung des Gewebes zu detektieren, wobei:
die Ultraschall-Diagnosevorrichtung dazu ausgelegt ist, eine Stoßwelle einer Ultraschallwelle zum Erzeugen einer Scherwelle durch das Subjekt zu senden und eine Verfolgungswelle einer Ultraschallwelle durch das Subjekt zu senden,
die Messeinheit (30) dazu ausgelegt ist, eine Phasenverschiebung des Gewebes nach der Erzeugung der Scherwelle auf der Basis des Empfangssignals der Ultraschallwelle zu messen, das durch die Verfolgungswelle gewonnen wird, und
die Fluktuations-Erfassungseinheit (40) dazu ausgelegt ist. die periodische Verschiebung nach der Erzeugung der Scherwelle zu erfassen,
wobei
die Ultraschall-Diagnosevorrichtung eine Scherwellengeschwindigkeits-Berechnungseinheit (50) enthält, die konfiguriert ist, um eine statistische Ausbreitungsgeschwindigkeit (Vs) der Scherwelle in dem Subjekt zu ermitteln, indem das Ergebnis der Verschiebungsmessung in einer Vielzahl von Tiefen nach der Erzeugung der Scherwelle, die durch die Messeinheit (30) gemessen wird, und das Erfassungsergebnis, das von der Fluktuations-Erfassungseinheit erfasst wurde, genutzt werden, und wobei ferner die Ultraschall-Diagnosevorrichtung konfiguriert ist, um:
- als eine Zeit-Raum-Karte Phasenverschiebungsdaten zu erzeugen, die eine Zeit entlang einer Achse und Positionen der Mehrzahl von Tiefen entlang der anderen Achse zeigen,
- ein Fluktuationsbande als einen Bereich zu bestimmen, der eine Vielzahl von Positionen umfasst, an denen eine Fluktuation in der Zeit-Raum-Karte detektiert wurde, wobei die Phasenverschiebung durch die Fluktuation in dem Fluktuationsband beeinflusst wird, und
- die statistische Ausbreitungsgeschwindigkeit (Vs) aus dem Ergebnis der Verschiebungsmessung in der Vielzahl von Tiefen, die das Fluktuationsband ausschließen, zu gewinnen.

2. Ultraschall-Diagnosevorrichtung nach Anspruch 1, wobei
die Messeinheit (30) dazu ausgelegt ist, auf der Basis des Empfangssignals an jedem Punkt in einer Vielzahl von Punkten im Subjekt die Verschiebung des Gewebes an den einzelnen Punkten zu messen und Verschiebungsdaten zu erzeugen, die die an der Vielzahl von Punkten gemessene Verschiebung über eine Vielzahl von Zeiten zeigen, und
die Fluktuationserfassungseinheit (40) dazu ausgelegt ist, auf der Basis der Verschiebungsdaten eine periodische Verschiebung unter den in der Vielzahl von Punkten gemessenen Verschiebungen zu erfassen.

3. Ultraschall-Diagnosevorrichtung nach Anspruch 2, wobei: die Fluktuationserfassungseinheit (40) dazu ausgelegt ist, auf der Basis der Verschiebungsdaten eine Frequenzanalyse einer zeitlichen Änderung der Verschiebung in jedem Punkt durchzuführen, und wenn eine maximale Frequenzkomponente enthalten ist, die die Beurteilungsbedingungen erfüllt, zu entscheiden, dass die Verschiebung an dem Punkt periodisch ist.

4. Ultraschall-Diagnosevorrichtung nach Anspruch 2 oder 3, wobei:
die Ultraschall-Diagnosevorrichtung dazu ausgelegt ist, ein Anzeigebild zu erzeugen, das Verschiebungen in einer Vielzahl von Punkten über eine Vielzahl von Zeiten auf der Basis der von der Messeinheit (30) erzeugten Verschiebungsdaten zeigt.

5. Ultraschall-Diagnosevorrichtung nach Anspruch 2, wobei:
die Ultraschall-Diagnosevorrichtung dazu ausgelegt ist, ein Anzeigebild zu erzeugen, das die einzelnen Punkte, in denen die periodischen Verschiebungen durch die Schwankungsdetektionseinheit detektiert wurden, unter der Vielzahl von Punkten, an denen die Verschiebungen durch die Messeinheit (30) gemessen wurden, anzeigt.

## Revendications

1. Dispositif de diagnostic à ultrasons, comprenant :
une unité (10, 12, 14) d'émission/réception qui est configurée pour délivrer en sortie un signal de transmission d'une onde ultrasonore et pour obtenir un signal de réception de l'onde ultrasonore depuis un sujet,
une unité (30) de mesure qui est configurée pour mesurer un déplacement d'un tissu dans le sujet sur la base du signal de réception de l'onde ultrasonore, et
une unité (40) de détection de fluctuation qui est configurée pour détecter un déplacement périodique sur la base du déplacement mesuré du tissu, dans lequel :
le dispositif de diagnostic à ultrasons est configuré pour transmettre une onde de poussée d'une onde ultrasonore pour générer une onde de cisaillement à travers le sujet et pour transmettre une onde de suivi d'une onde ultrasonore jusqu'au sujet,
l'unité (30) de mesure est configurée pour mesurer un décalage de phase du tissu après la génération de l'onde de cisaillement sur la base du signal de réception de l'onde ultrasonore qui est obtenu par l'onde de suivi, et
l'unité (40) de détection de fluctuation est configurée pour détecter le déplacement périodique après la génération de l'onde de cisaillement,
dans lequel le dispositif de diagnostic à ultrasons inclut une unité (50) de calcul de vitesse d'onde de cisaillement configurée pour obtenir une vitesse de propagation statistique (Vs) de l'onde de cisaillement dans le sujet en utilisant le résultat de mesure de déplacement à une pluralité de profondeurs après la génération de l'onde de cisaillement mesurée par l'unité (30) de mesure et le résultat de détection obtenu par l'unité de détection de fluctuation et en outre dans lequel le dispositif de diagnostic à ultrasons est configuré pour :
- générer, comme une carte temps-espace, des données de décalage de phase qui montrent un temps le long d'un axe et des positions de la pluralité de profondeurs le long de l'autre axe ;
- déterminer une bande de fluctuation comme une région qui comprend une pluralité de positions auxquelles une fluctuation a été détectée dans la carte temps-espace, dans lequel le décalage de phase est affecté par la fluctuation dans la bande de fluctuation, et
- obtenir la vitesse de propagation statistique (Vs) à partir du résultat de mesure de décalage à la pluralité de profondeurs à l'exclusion de la bande de fluctuation.

2. Dispositif de diagnostic à ultrasons selon la revendication 1, dans lequel :
l'unité (30) de mesure est configurée pour mesurer, à une pluralité de points dans le sujet, le déplacement du tissu aux points individuels sur la base du signal de réception à chaque point, et pour générer des données de déplacement montrant le déplacement mesuré à la pluralité de points sur une pluralité de temps, et
l'unité (40) de détection de fluctuation, sur la base des données de déplacement, est configurée pour détecter un déplacement périodique parmi les déplacements mesurés à la pluralité de points.

3. Dispositif de diagnostic à ultrasons selon la revendication 2, dans lequel :
l'unité (40) de détection de fluctuation est configurée pour exécuter une analyse de fréquence d'un changement temporel de déplacement à chaque point sur la base des données de déplacement, et si une composante fréquentielle maximum satisfaisant à des conditions de jugement est contenue, pour juger que le déplacement au point est périodique.

4. Dispositif de diagnostic à ultrasons selon la revendication 2 ou 3, dans lequel :
le dispositif de diagnostic à ultrasons est configuré pour former une image d'affichage qui montre des déplacements à une pluralité de points sur une pluralité de temps sur la base des données de déplacement générées par l'unité (30) de mesure.

5. Dispositif de diagnostic à ultrasons selon la revendication 2, dans lequel :
le dispositif de diagnostic à ultrasons est configuré pour former une image d'affichage qui spécifie les points individuels où les déplacements périodiques ont été détectés par l'unité de détection de fluctuation parmi la pluralité de points où les déplacements ont été mesurés par l'unité (30) de mesure.
